# EUROPEAN PATENT SPECIFICATION

(11) **EP 0 654 521 B1**
(45) Date of publication and mention of the grant of the patent: **15.09.1999**
(21) Application number: 94308424.4
(22) Date of filing: 15.11.1994
(51) Int. Cl.: C10G 11/18, C10G 57/00

(54) **Integrated catalytic cracking and olefin producing process**
Integriertes katalytisches Krack- und Olefinen Herstellungsverfahren
Procédé intégré de craquage catalytique et de production d'oléfines

(30) Priority: 19.11.1993 US 154831
(43) Date of publication of application: 24.05.1995
(73) Proprietor: Exxon Research and Engineering Company, (a Delaware corp.), Florham Park, New Jersey 07932 (US)
(72) Inventor: Kerby, Michael Charles, Jr., Baton Rouge, Louisiana 70810 (US); Bearden, Roby, Jr., Baton Rouge, Louisiana 70808 (US); Davis, Stephen Mark, Baton Rouge, Louisiana 70817 (US)
(74) Representative: Somers, Harold Arnold

(56) References cited:
- EP-A- 0 168 185
- EP-A- 0 259 156
- EP-A- 0 325 437
- EP-A- 0 577 280
- US-A- 3 894 935
- US-A- 4 968 401

## Description

This invention relates to a combined catalytic cracking and olefin producing process.

The emergence of low emissions fuels has created a need to increase the availability of olefins for use in alkylation, oligomerization, MTBE and ETBE synthesis. In addition, a low cost supply of olefins continues to be in demand to serve as feedstock for polyolefin production.

Fixed bed processes for light paraffin dehydrogenation have recently attracted renewed interest for increasing olefin production. However, these type of processes typically require a high capital investment as well as a high operating cost. It is, therefore, advantageous to increase olefin yield using processes which require only a minimal amount of capital investment. It would be particularly advantageous to increase olefin yield in catalytic cracking processes.

US-A-4,830,728 discloses a fluid catalytic cracking (FCC) unit which is operated to maximize olefin production. The FCC unit has two separate risers in which different feed streams are introduced. The operation of the risers is designed so that a certain catalyst will act to convert a heavy gas oil in one riser and a different catalyst will act to crack a lighter olefin/naphtha feed in the other riser. Conditions within the heavy gas oil riser are modified to maximize either gasoline or olefin production. The primary means of maximizing production of the desired product is by using a specified catalyst.

A problem inherent in producing olefin products using FCC units is that the process depends upon a specific catalyst balance to maximize production. In addition, even if a specific catalyst balance can be maintained to maximize overall olefin production, olefin selectivity is generally low due to undesirable side reactions such as extensive cracking, isomerization, aromatization and hydrogen transfer reactions. It is, therefore, desirable that olefin production be maximized in a process which allows a high degree of control over olefin selectivity.

EP-A-0325437 describes and claims a process for regenerating a coke-contaminated fluid cracking catalyst in a regeneration zone at a pressure in the range from above 240 kPa to 446 kPa and a temperature in the range from 650°C to 815°C while injecting the regeneration zone with enough oxygen-containing regeneration gas to maintain a dense fluid bed of regeneration catalyst, and regenerate the catalyst before returning it to a fluid cracker, comprising,
a) withdrawing a controlled stream of the regenerator catalyst and introducing it into a dehydrogenation zone at a temperature below those prevailing in the regeneration zone, the dehydrogenation zone being located in a catalyst cooler, externally relative to the cracker and regenerator, the amount of the stream being sufficient to supply the endothermic heat of reaction for dehydrogenation of alkanes in the dehydrogenation zone,
b) introducing a feedstream of the alkanes into the dehydrogenation zone in an amount sufficient to maintain hot withdrawn catalyst in a state of fluidization in the catalyst cooler, the state of fluidization existing in a sub-transport regime while maintained at a temperature high enough to convert at least 50% of the alkanes, and concurrently to cool the catalyst,
c) transporting the cooled catalyst from the dehydrogenation zone, the catalyst now at a temperature in the range from 650 to 731°C, to the regeneration zone, and mixing hot catalyst therein with the cooled catalyst; and
d) withdrawing products of dehydrogenation in an effluent stream from the catalyst cooler.

In one embodiment, the process comprises withdrawing a controlled stream of spent catalyst from the fluid cracker and introducing the spent catalyst directly into the dehydrogenation zone, and transporting the cooled catalyst for flow-controlled introduction into a riser of the fluid cracker, in the lower portion thereof, and in addition, introducing a minor amount relative to the alkanes, of steam into the dehydrogenation zone, the amount being sufficient, in combination with the alkanes to strip hydrocarbons remaining in the spent catalyst.

In order to overcome problems inherent in the prior art, the present invention provides an integrated catalytic cracking and alkane dehydrogenation process according to claim 1.

The catalytic cracking catalyst may comprise a zeolite crystalline framework oxide.

The alkane feed may comprise at least one component selected from ethane, propane, butane, pentane, hexane, heptane, octane, nonane, decane, isobutane, isopentanes, isohexanes, isoheptanes and iso-octanes.

Coke may be deposited onto the regenerated catalytic cracking catalyst by adding a coke precursor to the regenerated catalytic cracking catalyst under dehydrogenation conditions. Coke may be deposited onto the regenerated catalytic cracking catalyst to obtain a dehydrogenation catalyst which comprises from about 0.2-10 wt% carbon.

The alkane feed may be dehydrogenated to an olefin product which comprises at least 1 wt% total olefin.

The alkane feed may be dehydrogenated with the dehydrogenation catalyst at an alkane vapor residence time in the range of from about 0.5-10 seconds.

The regenerated catalyst of step (e) may be partially or incompletely regenerated catalyst.

### BRIEF DESCRIPTION OF THE DRAWING

An illustrative embodiment of the invention, given by way of non-limitative example only, is now described with reference to the drawing wherein:

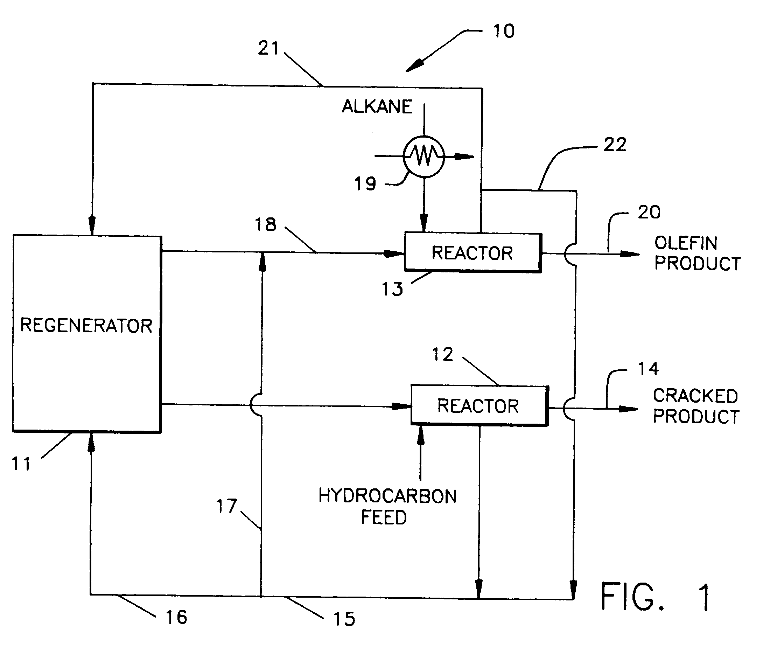

Fig. 1 is a schematic representation of the said embodiment of the invention.

### DETAILED DESCRIPTION OF THE INVENTION

Catalytic cracking is a process which is well known in the art of petroleum refining and generally refers to converting a large hydrocarbon molecule to a smaller hydrocarbon molecule by breaking at least one carbon to carbon bond. For example, large paraffin molecules can be cracked to a paraffin and an olefin, and a large olefin molecule can be cracked to two or more smaller olefin molecules. Long side chain molecules which may be present on aromatic rings or naphthenic rings can also be cracked.

It has been found that a coked catalytic cracking catalyst can be used to enhance the dehydrogenation of an alkane feed stream to produce an olefin stream. By using a coked catalytic cracking catalyst as the catalyst for the dehydrogenation reaction, this aspect of the invention can be integrated into the catalytic cracking process to increase olefin yield in the overall reaction scheme. This increased olefin yield is advantageous since the olefin product can be used as a feedstock in other reaction processes to either increase the octane pool in a refinery, or the olefins can be used in the manufacture of gasoline additives which are required to reduce undesirable hydrocarbon emissions. In addition, the process of this invention allows for high olefin selectivity such that a portion of the olefin stream can also be used in other chemicals processes such as polyolefin production.

In the catalytic cracking step of this invention, the hydrocarbon feed is preferably a petroleum hydrocarbon. The hydrocarbon is preferably a distillate fraction having an initial ASTM boiling range of about 400°F (204.4°C). Such hydrocarbon fractions include gas oils, thermal oils, residual oils, cycle stocks, topped whole crudes, tar sand oils, shale oils, synthetic fuels, heavy hydrocarbon fractions derived from the destructive hydrogenation of coal, tar, pitches, asphalts, and hydrotreated feed stocks derived from any of the foregoing.

The hydrocarbon feed is preferably introduced into a riser which feeds a catalytic cracking reactor vessel. Preferably, the feed is mixed in the riser with catalytic cracking catalyst that is continuously recycled.

The hydrocarbon feed can be mixed with steam or an inert type of gas at such conditions so as to form a highly atomized stream of a vaporous hydrocarbon-catalyst suspension. Preferably, this suspension flows through the riser into the reactor vessel. The reactor vessel is preferably operated at a temperature of about 800-1200°F (426.7 to 648.9°C) and a pressure of about 0-100 psig (1.014 to 7.910 bar).

The catalytic cracking reaction is essentially quenched by separating the catalyst from the vapor. The separated vapor comprises the cracked hydrocarbon product, and the separated catalyst comprises a carbonaceous material (i.e., coke) as a result of the catalytic cracking reaction.

The coked catalyst is preferably recycled to contact additional hydrocarbon feed after the coke material has been removed. Preferably, the coke is removed from the catalyst in a regenerator vessel by combusting the coke from the catalyst. Preferably, the coke is combusted at a temperature of about 900-1400°F (482.2-760°C) and a pressure of about 0-100 psig (1.014 to 7.910 bar). After the combustion step, the regenerated catalyst is recycled to the riser for contact with additional hydrocarbon feed.

The catalyst which is used in this invention can be any catalyst which is typically used to catalytically "crack" hydrocarbon feeds. It is preferred that the catalytic cracking catalyst comprise a crystalline tetrahedral framework oxide component. This component is used to catalyze the breakdown of primary products from the catalytic cracking reaction into clean products such as naphtha for fuels and olefins for chemical feedstocks. Preferably, the crystalline tetrahedral framework oxide component is selected from the group consisting of zeolites, tectosilicates, tetrahedral aluminophophates (ALPOs) and tetrahedral silicoaluminophosphates (SAPOs). More preferably, the crystalline framework oxide component is a zeolite.

Zeolites which can be employed in accordance with this invention include both natural and synthetic zeolites. These zeolites include gmelinite, chabazite, dachiardite, clinoptilolite, faujasite, heulandite, analcite, levynite, erionite, sodalite, cancrinite, nepheline, lazurite, scolecite, natrolite, offretite, mesolite, mordenite, brewsterite, and ferrierite. Included among the synthetic zeolites are zeolites X, Y, A, L, ZK-4, ZK-5, B, E, F, H, J, M, Q, T, W, Z, alpha and beta, ZSM-types and omega.

In general, aluminosilicate zeolites are effectively used in this invention. However, the aluminum as well as the silicon component can be substituted for other framework components. For example, the aluminum portion can be replaced by boron, gallium, titanium or trivalent metal compositions which are heavier than aluminum. Germanium can be used to replace the silicon portion.

The catalytic cracking catalyst used in this invention can further comprise an active porous inorganic oxide catalyst framework component and an inert catalyst framework component. Preferably, each component of the catalyst is held together by attachment with an inorganic oxide matrix component.

The active porous inorganic oxide catalyst framework component catalyzes the formation of primary products by cracking hydrocarbon molecules that are too large to fit inside the tetrahedral framework oxide component. The active porous inorganic oxide catalyst framework component of this invention is preferably a porous inorganic oxide that cracks a relatively large amount of hydrocarbons into lower molecular weight hydrocarbons as compared to an acceptable thermal blank. A low surface area silica (e.g., quartz) is one type of acceptable thermal blank. The extent of cracking can be measured in any of various ASTM tests such as the MAT (microactivity test, ASTM # D3907-8). Compounds such as those disclosed in Greensfelder, B. S., et al., Industrial and Engineering Chemistry, pp. 2573-83, Nov. 1949, are desirable. Alumina, silica-alumina and silica-alumina-zirconia compounds are preferred.

The inert catalyst framework component densifies, strengthens and acts as a protective thermal sink. The inert catalyst framework component used in this invention preferably has a cracking activity that is not significantly greater than the acceptable thermal blank. Kaolin and other clays as well as α-alumina, titania, zirconia, quartz and silica are examples of preferred inert components.

The inorganic oxide matrix component binds the catalyst components together so that the catalyst product is hard enough to survive interparticle and reactor wall collisions. The inorganic oxide matrix can be made from an inorganic oxide sol or gel which is dried to "glue" the catalyst components together. Preferably, the inorganic oxide matrix will be comprised of oxides of silicon and aluminum. It is also preferred that separate alumina phases be incorporated into the inorganic oxide matrix. Species of aluminum oxyhydroxides-γ-alumina, boehmite, diaspore, and transitional aluminas such as α-alumina, β-alumina, γ-alumina, δ-alumina, ε-alumina, κ-alumina, and ρ-alumina can be employed. Preferably, the alumina species is an aluminum trihydroxide such as gibbsite, bayerite, nordstrandite, or doyelite.

According to this invention, in order to produce an olefin stream, an olefin reaction is commenced by contacting an alkane feed stream with a coked catalytic cracking catalyst. The alkane feed stream of this invention is preferably a C₂-C₁₀ alkane composition. The alkane composition can be either branched or unbranched. Such alkane feed may comprise at least one component selected from ethane, propane, butane, pentane, hexane, heptane, octane, nonane, decane, isobutane, isopentanes, isohexanes, isoheptanes and iso-octanes.

A coked catalytic cracking catalyst can be obtained by any of numerous means of controlling catalyst regeneration. Such means are the subject-matter of further applications EP-A-0 654 519, EP-A-0 654 520, EP-A-0 654 522 and EP-A-0 654 523, all having the same filing date. As one example, the coked catalytic cracking catalyst can be obtained as a result of a partial or incomplete regeneration of at least a portion of the spent catalyst stream in a FCC unit. One of ordinary skill in the art will be able to attain the desired concentration of coke on the catalytic cracking catalyst using well known means of adjusting temperature, oxygen content or burn time within the regenerator portion of the FCC unit.

The conversion of alkane to olefin in this invention generally involves a dehydrogenation reaction. In the dehydrogenation reaction, alkanes are converted to olefins and molecular hydrogen. This reaction is highly endothermic. Preferably, the dehydrogenation reaction is carried out at a temperature of about 800-1600°F (426.7-871.1°C), more preferably about 800-1400°F (426.7 to 760.0°C).

The dehydrogenation reaction is somewhat dependent upon pressure. In general, the higher the pressure, the lower the conversion of alkane to olefin. Preferably, the process is carried out at about 0-100 psig (1.014 to 7.910 bar).

The contact time between the alkane stream and the coked catalytic cracking catalyst will also affect the yield of olefin product. Typically, optimal contact between the coked catalyst and the alkane stream is attained when the olefin product stream contains a concentration of at least about 1 wt % total olefin. Preferably, alkane vapor residence time will range from about 0.5-60 seconds, more preferably, about 1.0-10 seconds.

A preferred embodiment of this invention is shown in Fig. 1 in which the dehydrogenation reaction is incorporated into a catalytic cracking process. In the preferred embodiment, regenerated catalytic cracking catalyst particles and a portion of the catalytic cracking catalyst particles which become spent from the cracking reaction are combined. The combination of catalyst particles is such that the overall catalyst composition is effectively coked to provide an active dehydrogenation catalyst. It is preferred that the regenerated catalytic cracking particles and the spent catalytic cracking particles be combined so that the heat content of the combined composition is in the preferred range for the dehydrogenation reaction.

In the preferred embodiment of Fig. 1, the integrated catalytic cracking and alkane dehydrogenation process takes place generally in a FCC unit 10 which includes a regenerator 11, a cracking reactor 12 and a satellite reactor 13. The cracking reactor 12 comprises a main reactor vessel and can include a riser conduit where hydrocarbon feed is injected and initially contacts regenerated catalytic cracking catalyst from the regenerator 11. The catalytic cracking reaction is initiated as the hydrocarbon feed contacts the catalyst, and continues until the catalyst is separated from the hydrocarbon within the cracking reactor 12. Separation can be accomplished using any of the acceptable FCC separation devices such as cyclone separators. After separation, the cracked hydrocarbon product leaves the reactor 12 through a product line 14. The separated catalyst, which has become coked (i.e, spent) in the cracking reaction, leaves the reactor 12 through a recycle line 15 where the catalyst is then divided into a first and second portion. The first catalyst portion is sent to the regenerator 11 through a regenerator supply line 16, and the second catalyst portion is sent to a regenerator by-pass line 17.

Coke is removed from the first portion of the spent catalyst in the regenerator 11 under conventional regeneration conditions. The coke is effectively removed by combusting the catalyst until it is sufficiently active to promote the hydrocarbon cracking reaction. Preferably, the regenerated catalyst will contain no more than about 0.5 wt % coke, more preferably the regenerated catalyst will contain no more than about 0.2 wt % coke.

A portion of the regenerated catalyst is recycled to the cracking reactor 12 where additional hydrocarbon feed is injected and cracked. In addition, a second portion of the regenerated catalyst is sent through feed line 18 to the satellite reactor 13. The satellite reactor 13 can be any type of reactor vessel that is operable under dehydrogenation conditions. For example, the satellite reactor 13 can be a transfer line riser reactor, a slumped bed reactor, a spouting bed reactor or a moving bed reactor. Preferably, the satellite reactor 13 will be capable of supporting a fluid bed catalyst at a density of about 1-45 Ibs of catalyst per cubic foot of reactor volume.

Prior to entering the satellite reactor 13, the regenerated catalyst is combined with the spent catalyst from the regenerator by-pass line 17. This combination of regenerated and spent catalyst forms a dehydrogenation catalyst which is fed to the satellite reactor 13 through a satellite reactor feed line 18. As the dehydrogenation catalyst is introduced to the satellite reactor 13, it is contacted with an alkane stream to commence the dehydrogenation reaction.

Typically, the regenerated catalyst that is combined with the spent catalyst in the satellite reactor feed line 18 will contain sufficient heat from the regenerator 11 to maintain the desired dehydrogenation temperature in the satellite reactor 13. It may be desirable, however, to add heat to the dehydrogenation catalyst to achieve the desired dehydrogenation conditions in the satellite reactor 13. In this case, the alkane stream can be preheated, for example by adding a heater 19.

The dehydrogenation reaction is effectively quenched by separating the dehydrogenated products from the catalyst. Separation can be accomplished using any of the acceptable FCC separation type devices such as cyclone separators. After separation, the dehydrogenation product leaves the satellite reactor 13 through a dehydrogenation product line 20, and the separated catalyst, which becomes further coked during the dehydrogenation reaction, is returned to the regenerator 11 through a spent catalyst line 21 or is combined with the recycle line 15 by a line 22.

The following Example, which is not an embodiment of the invention, illustrates the suitability of partially-coked cracking catalysts for use in dehydrogenating alkanes to yield olefin-containing products.

### EXAMPLE

An equilibrium zeolite beta FCC catalyst (SiO₂ 65.1 wt %; Al₂O₃ wt %; Na₂O 0.28 wt %; REO₂ 2.14 wt %) was placed in a fixed bed quartz reactor. The temperature of the reactor was maintained at 1250°F (676.7°C), and the pressure was maintained at 0 psig (1.014 bar). Six runs were made varying the total carbon content on the catalyst from 0.2 wt % to 2.7 wt %. The catalyst in runs 2-6 was pretreated with a hydrocarbon to increase the base level carbon content, thereby representing a dehydrogenation catalyst useful in the process of this invention. Iso-butane feed was passed through the reactor at 1 second residence time and GHSV of 1066. The results are shown in Table 1.

**Table 1**

| Run Number | 001 | 002 | 003 | 004 | 005 | 006 |
|---|---|---|---|---|---|---|
| Feed Pre-Treat | none Resid | HCN | HCN | Resid | Resid | |
| Cat/Oil Pre-Treat | --- | 5.1 | 3.0 | 4.8 | 3.0 | 1.8 |
| Carbon Content (wt%) | 0.2 | 0.8 | 1.1 | 2.2 | 2.5 | 2.7 |
| | | | | | | |
| Feed | i-C₄H₁₀ | i-C₄H₁₀ | i-C₄H₁₀ | i-C₄H₁₀ | i-C₄H₁₀ | i-C₄H₁₀ |
| | | | | | | |
| Iso-C₄H₁₀ Conversion (wt%) | 45.3 | 37.8 | 39.4 | 33.1 | 34.3 | 36.0 |
| | | | | | | |
| Selectivity (%) | | | | | | |
| C₁-C₃ | 55.1 | 43.8 | 41.7 | 35.0 | 35.6 | 36.2 |
| n-C₄H₁₀ | 3.0 | 0.3 | 2.2 | 1.8 | 1.8 | 2.0 |
| 1-C₄H₈ | 5.6 | 7.0 | 6.3 | 5.6 | 5.8 | 5.8 |
| t-2-C₄H₈ | 5.9 | 6.9 | 6.3 | 5.6 | 5.6 | 5.8 |
| c-2-C₄H₈ | 5.3 | 5.6 | 5.1 | 4.5 | 4.6 | 4.6 |
| Iso-C₄H₈ | 20.8 | 31.1 | 36.4 | 45.5 | 45.1 | 44.0 |
| >C4's | 4.4 | 5.5 | 2.1 | 1.4 | 1.5 | 1.6 |
| | | | | | | |
| Iso-C₄H₈ Yield (wt%) | 9.4 | 11.7 | 14.3 | 15.0 | 15.5 | 15.8 |

Having now fully described this invention, it will be appreciated by those skilled in the art that the invention can be performed within a wide range of parameters within what is claimed in the claims which follow.

## Claims

1. An integrated catalytic cracking and alkane dehydrogenation process comprising the steps of:
(a) catalytically cracking a petroleum hydrocarbon feedstock employing a catalytic cracking catalyst, and obtaining coked catalytic cracking catalyst and cracked hydrocarbon product;
(b) regenerating coked catalytic cracking catalyst from step (a) to form regenerated catalytic cracking catalyst, and employing regenerated catalyst for the catalytic cracking of further amounts of hydrocarbon feedstock;
characterised by :
(c) dividing coked catalytic cracking catalyst obtained from step (a) into at least first and second portions;
(d) regenerating the first portion, and employing the regenerated first portion for the catalytic cracking of further amounts of hydrocarbon feedstock (as specified in step (b) hereof);
(e) combining some regenerated catalytic cracking catalyst with the second portion of coked catalytic cracking catalyst to form a dehydrogenation catalyst; and
(f) dehydrogenating a feed comprising one or more C₂-C₁₀ alkanes employing the dehydrogenation catalyst obtained by step (e).

2. The process of claim 1, wherein the catalytic cracking catalyst comprises a zeolite crystalline framework oxide.

3. The process of claim 1 or claim 2, wherein the alkane feed comprises at least one component selected from ethane, propane, butane, pentane, hexane, heptane, octane, nonane, decane, isobutane, isopentanes, isohexanes, isoheptanes and iso-octanes.

4. The process of any preceding claim, wherein coke is deposited onto the regenerated catalytic cracking catalyst by adding a coke precursor to the regenerated catalytic cracking catalyst under dehydrogenation conditions.

5. The process of claim 4, wherein coke is deposited onto the regenerated catalytic cracking catalyst to obtain a dehydrogenation catalyst which comprises about 0.2-10 wt% carbon.

6. The process of any preceding claim, wherein the alkane feed is dehydrogenated to an olefin product which comprises at least 1 wt% total olefin.

7. The process of any preceding claim, wherein the alkane feed is dehydrogenated with the dehydrogenation catalyst at an alkane vapor residence time of about 0.5-10 seconds.

8. The process of any preceding claim wherein coked catalyst is obtained as a result of a partial or incomplete regeneration of at least a portion of the spent catalyst stream in a fluid catalytic cracking unit.

## Patentansprüche

1. Integriertes Verfahren zum katalytischen Cracken und zur Alkandehydrierung, bei dem:
(a) ein Erdölkohlenwasserstoffeinsatzmaterial unter Verwendung eines katalytischen Crackkatalysators katalytisch gecrackt wird und ein gebrauchter katalytischer Crackkatalysator und gecracktes Kohlenwasserstoffprodukt erhalten werden,
(b) gebrauchter katalytischer Crackkatalysator aus Schritt (a) unter Bildung von regeneriertem katalytischem Crackkatalysator regeneriert wird und regenerierter Katalysator für das katalytische Cracken weiterer Mengen Kohlenwasserstoffeinsatzmaterial verwendet wird,
dadurch gekennzeichnet, daß
(c) gebrauchter katalytischer Crackkatalysator, der aus Schritt (a) erhalten wurde, in mindestens einen ersten und einen zweiten Teil aufgeteilt wird,
(d) der erste Teil regeneriert wird und der regenerierte erste Teil für das katalytische Cracken weiterer Mengen Kohlenwasserstoffeinsatzmaterial (wie in Schritt (b) hiervon spezifiziert) verwendet wird,
(e) etwas regenerierter katalytischer Crackkatalysator mit dem zweiten Teil des gebrauchten katalytischen Crackkatalysators unter Bildung eines Dehydrierungskatalysators kombiniert wird und
(f) ein Einsatzmaterial, das ein oder mehrere C₂-C₁₀ Alkane umfaßt, unter Verwendung des Dehydrierungskatalysators, der in Schritt (e) erhalten wurde, dehydriert wird.

2. Verfahren nach Anspruch 1, bei dem der katalytische Crackkatalysator kristallines Zeolitgerüstwerkoxid umfaßt.

3. Verfahren nach Anspruch 1 oder Anspruch 2, bei dem das Alkaneinsatzmaterial mindestens eine Komponente ausgewählt aus Ethan, Propan, Butan, Pentan, Hexan, Heptan, Octan, Nonan, Decan, Isobutan, Isopentanen, Isohexanen, Isoheptanen und Isooctanen umfaßt.

4. Verfahren nach einem der vorhergehenden Ansprüche, bei dem Koks auf dem regenerierten katalytischen Crackkatalysator aufgebracht wird, indem ein Koksvorläufer zu dem regenerierten katalytischen Crackkatalysator unter Dehydrierungsbedingungen zugesetzt wird.

5. Verfahren nach Anspruch 4, bei dem Koks auf dem regenerierten katalytischen Crackkatalysator aufgebracht wird, um einen Dehydrierungskatalysator zu erhalten, der etwa 0,2 bis 10 Gew.-% Kohlenstoff umfaßt.

6. Verfahren nach einem der vorhergehenden Ansprüche, bei dem das Alkaneinsatzmaterial zu einem Olefinprodukt dehydriert wird, das mindestens 1 Gew.-% Olefin umfaßt.

7. Verfahren nach einem der vorhergehenden Ansprüche, bei dem das Alkaneinsatzmaterial mit dem Dehydrierungskatalysator mit einer Alkandampfverweilzeit von etwa 0,5 - 10 Sekunden dehydriert wird.

8. Verfahren nach einem der vorhergehenden Ansprüche, bei dem verkokter Katalysator als Resultat einer partiellen oder unvollständigen Regenerierung von mindestens einem Teil des verbrauchten Katalysatorstroms in einer katalytischen Wirbelschicht-Crackanlage erhalten wird.

## Revendications

1. Procédé intégré de craquage catalytique et de déshydrogénation d'alcanes, comprenant les étapes consistant :
(a) à craquer catalytiquement une charge d'hydrocarbures de pétrole en utilisant un catalyseur de craquage catalytique et à obtenir un catalyseur de craquage catalytique cokéfié et un produit hydrocarboné craqué,
(b) à régénérer le catalyseur de craquage catalytique cokéfié de l'étape (a) pour former un catalyseur de craquage catalytique régénéré et à utiliser le catalyseur régénéré pour le craquage catalytique de quantités supplémentaires de charge d'hydrocarbures,
caractérisé par les étapes suivantes :
(c) on scinde le catalyseur de craquage catalytique cokéfié obtenu en l'étape (a) en au moins une première et une seconde parties,
(d) on régénère la première partie et on utilise la première partie régénérée pour le craquage catalytique de quantités supplémentaires de charge d'hydrocarbures (comme spécifié en l'étape (b)),
(e) on combine une partie du catalyseur de craquage catalytique régénéré avec la seconde partie du catalyseur de craquage catalytique cokéfié pour former un catalyseur de déshydrogénation et
(f) on déshydrogène une charge d'alimentation comprenant un ou plusieurs alcanes en C₂-C₁₀ en utilisant le catalyseur de déshydrogénation obtenu à l'étape (e).

2. Procédé selon la revendication 1, dans lequel le catalyseur de craquage catalytique comprend un oxyde à ossature cristalline zéolitique.

3. Procédé selon la revendication 1 ou 2, dans lequel la charge d'alimentation en alcanes comprend au moins un composant choisi parmi l'éthane, le propane, le butane, le pentane, l'hexane, le heptane, l'octane, le nonane, le décane, l'isobutane, les isopentanes, les isohexanes, les isoheptanes et les iso-octanes.

4. Procédé selon l'une quelconque des revendications précédentes, dans lequel du coke se dépose sur le catalyseur de craquage catalytique régénéré en ajoutant un précurseur de coke au catalyseur de craquage catalytique régénéré dans des conditions de déshydrogénation.

5. Procédé selon la revendication 4, dans lequel du coke se dépose sur le catalyseur de craquage catalytique régénéré pour obtenir un catalyseur de déshydrogénation qui comprend environ 0,2% à 10% en poids de carbone.

6. Procédé selon l'une quelconque des revendications précédentes, dans lequel la charge d'alimentation d'alcanes est déshydrogénée en un produit oléfinique qui comprend au moins 1% en poids total d'oléfines.

7. Procédé selon l'une quelconque des revendications précédentes, dans lequel la charge d'alimentation d'alcanes est déshydrogénée avec le catalyseur de déshydrogénation pendant une durée de séjour des vapeurs d'alcanes dans la plage d'environ 0,5 à 10 secondes.

8. Procédé selon l'une quelconque des revendications précédentes, dans lequel le catalyseur cokéfié est obtenu comme le résultat d'une régénération partielle ou incomplète d'au moins une partie du courant de catalyseur utilisé dans une unité de craquage catalytique liquide.
